Europäisches Patentamt

European Patent Office

Office européen des brevets

(11) Veröffentlichungsnummer: **0 274 587 B1**

## (12) EUROPÄISCHE PATENTSCHRIFT

(45) Veröffentlichungstag der Patentschrift: **22.04.92**

(21) Anmeldenummer: **87115936.4**

(22) Anmeldetag: **30.10.87**

(51) Int. Cl.5: **C02F 3/10**, C12N 11/14, C02F 3/12, C02F 3/28, C02F 3/30

(54) **Biotechnologische Verfahren unter Verwendung von immobilisierten Microorganismen.**

(30) Priorität: **15.11.86 DE 3639153**

(43) Veröffentlichungstag der Anmeldung:
**20.07.88 Patentblatt 88/29**

(45) Bekanntmachung des Hinweises auf die
Patenterteilung:
**22.04.92 Patentblatt 92/17**

(84) Benannte Vertragsstaaten:
**AT BE CH FR GB LI LU NL SE**

(56) Entgegenhaltungen:
**EP-A- 175 568        DE-A- 2 658 894
DE-A- 3 105 768        DE-A- 3 410 650
FR-A- 2 161 240        GB-A- 2 004 300**

**LEHR- UND HANDBUCH DER ABWASSER-
TECHNIK, Auflage 3, Seiten 95-97, Ernst &
Sohn Verlag für Architektur und technische
Wissenschaften, Berlin, DE; Band IV:
Biologisch-chemische und weitergehende
Abwasserreinigung**

(73) Patentinhaber: **Schott Glaswerke
Hattenbergstrasse 10**

**W-6500 Mainz(DE)**

(84) Benannte Vertragsstaaten:
**BE CH FR LI LU NL SE AT**

(73) Patentinhaber: **CARL-ZEISS-STIFTUNG
Schott Glaswerke Hattenbergstrasse 10
W-6500 Mainz 1(DE)**

(84) Benannte Vertragsstaaten:
**GB**

(72) Erfinder: **Trösch, W., Dr.
Egerweg 35
D-7000 Stuttgart 61(KI)**
Erfinder: **Kiefer, Werner, Dr.
Jupiterweg 19
W-6500 Mainz 21(DE)**
Erfinder: **Lohmann, Karlheinz
Sertoriusring 9
W-6500 Mainz 21(DE)**
Erfinder: **Dürolf, Hans
Sertoriusring 11
W-6500 Mainz 21(DE)**

EP 0 274 587 B1

(74) Vertreter: **Dr. Fuchs, Dr. Luderschmidt
Dipl.-Phys. Seids, Dr. Mehler Patentanwälte
Abraham-Lincoln-Strasse 7
W-6200 Wiesbaden(DE)**

# Beschreibung

Die Erfindung betrifft ein Verfahren zur Durchführung biotechnologischer Prozesse unter Verwendung von Bioreaktoren im Fest- oder Wirkelbettbetrieb gemäß dem Oberbegriff des Anspruchs 1.

Das verwendete Trägermaterial ist aus der DE-A 34 10 650 bekannt. Es ermöglicht hohe Raum-Zeit-Ausbeuten (Vermehrungsraten pro Volumen- und Zeiteinheit) sowohl bei aeroben als auch bei anaeroben Prozessen der Biotechnologie.

Bei anaeroben Systemen, bei denen die erzielbaren Vermehrungsgeschwindigkeiten und Konzentrationen der Biomasse erheblich kleiner sind als bei aeroben Systemen, ist es im allgemeinen erforderlich, die Biomasse im Reaktor zurückzuhalten und aufzukonzenzentrieren. Dafür ist in der Regel ein Festbett-Umlaufreaktor gut geeignet. Darin ist das Trägermaterial, in welchem die Mikroorganismen immobilisiert sind, weitgehend ortsfest, und eine den Stoffaustausch bewirkende Flüssigkeit strömt an dem Trägermaterial vorbei. Dadurch können die Mikroorganismen sich ungestört vermehren, und es können auch bei anaeroben Prozessen verhältnismäßig hohe Raum-Zeit-Ausbeuten erzielt werden. Ein wesentlicher Nachteil dieses Verfahrens besteht jedoch darin, daß die Flüssigkeit im wesentlichen frei von Feststoffen sein muß. Außerdem muß der Bioreaktor in bestimmten Zeitabständen gereinigt werden, da sonst leicht eine Verstopfung des Festbettes mit Mikroorganismen, Reaktionsprodukten oder organischen Verbindungen wie zum Beispiel Metallsulfiden eintreten kann. Schließlich besteht bei einem Festbett-Reaktor grundsätzlich der Nachteil, daß der Stoffaustausch langsam und ungleichmäßig vor sich geht.

Bei aeroben Prozessen liegt meist ein rasches mikrobielles Wachstum vor, so daß in der Regel ständig ein Teil der Biomasse aus dem Bioreaktor entfernt werden muß, zum Beispiel durch Flotation, um in dem Bioreaktor eine gewünschte Konzentration der Biomasse konstant zu halten. Außerdem müssen große Mengen Nährstoffe und Abbauprodukte rasch transportiert werden. Es ist deshalb bekannt, Wirbelbett-Bioreaktoren zu verwenden; bei diesen erwartet man wegen der ständigen Bewegung der Träger und der als Wirbelmedium dienenden Nährlösung eine gute Versorgung der Mikroorganismen mit Nährstoffen und bei aeroben Prozessen mit Sauerstoff. Um diese erwartete bessere Versorgung (und Entsorgung) weitergehend auszunutzen, ist es ferner dabei bekannt geworden, poröses Trägermaterial zu verwenden und dadurch die Menge Mikroorganismen pro Trägerkörper zu vergrößern (DE-A 34 10 650). Es hat sich jedoch gezeigt, daß die Erhöhung des Stoffwechsel-Umsatzes geringer war als erwartet, und daß die Trägerkörper leicht durch Überwachsen mit Biomasse verschleimten; dies wirkte sich gleichfalls ungünstig auf den erzielbaren Stoffwechsel-Umsatz aus.

Die Erfindung geht von der Aufgabe aus, ein Verfahren für Bioreaktorprozesse im Fest- und Wirbelbettbetrieb zur Verfügung zu stellen, in dem Mikroorganismen oder tierische Zellen in einer hohen Volumenkonzentration immobilisiert werden können und der Bioreaktor mit stark verminderter Verstopfungsgefahr betrieben werden kann.

Diese Aufgabe wird nach der Erfindung mit den kennzeichnenden Merkmale nach Anspruch 1 gelöst.

Mit dem erfindungsgemäßen verfahren ist es möglich, die Vorteile des Festbettreaktors mit den Vorteilen des Wirbelbettreaktors zu vereinigen. Die in einem Wirbelbett unübliche Verwendung von kugelförmigen Sinter-Trägerkörpern macht es möglich, durch passende Wahl von Durchmesser und Dichte der Sinterkörper die antreibende Wirkung des Wirbelmediums so zu verringern, daß eine hohe Relativgeschwindigkeit zwischen der Sinterkörper-Oberfläche und der Wirbelströmung vorliegt und dadurch sowohl ein rascher Stoffaustausch mit hohem Konzentrationsgefälle als auch ein Abscheren herausgewachsener Biomasse erzielt wird, wobei das Abscheren durch die Kontakte der Kugeln untereinander wirkungsvoll unterstützt wird und gleichmäßig sowie vollständig erfolgt. Dies gilt besonders dann, wenn in dem erfindungsgemäßen Trägermaterial Sinterkörper verhältnismäßig hoher Dichte vorliegen, wie sie ohne weiteres aus Sinterglas hergestellt werden können. Hinzu kommt der Vorteil, daß Kugeln ein in bezug auf ihre Oberfläche maximales Volumen haben, so daß verhältnismäßig viel Mikroorganismen und/oder Zellen in den offenen Poren der kugelförmigen Sinterkörper immobilisiert werden können und sich auch bei Verwendung der Sinterkörper in einem Wirbelbett die Mikroorganismen oder Zellen ähnlich wie in einem Festbettreaktor ungestört vermehren können, so daß auch langsam wachsende Mikroorganismen gute Lebensbedingungen vorfinden. Durch den erwähnten guten Stoffaustausch mit der Umgebung ist eine ausreichende Ernährung und Entsorgung der Mikroorganismen oder Zellen auch bei verhältnismäßig großen Konzentrationen und Kugeldurchmessern ohne weiteres sicherzustellen. Im Lichte der vorliegenden Erfindung scheinen die beschriebenen Nachteile bekannter Wirbelbett-Bioreaktoren vorwiegend darauf zurückzuführen, daß bei den üblichen nicht kugelförmigen Trägerkörpern im Wirbelbett in unmittelbarer Nähe der Trägerkörper-Oberflächen die Relativgeschwindigkeit zwischen der Oberfläche und dem Flüssigkeitsstrom verhältnismäßig klein sein kann, so daß dort eine starke Abreicherung von Nährstoffen und Sauerstoff eintreten kann. Dadurch wird die Raum-Zeit-Ausbeute verringert.

Es ist bekannt, in einem Träger für die Immobilisierung von bioaktiven Materialien ein mit bestimmten polymeren Kunststoffen beschichtetes Grundmaterial zu verwenden, das unter anderem in Form von Kügelchen vorliegen kann und aus porösem oder nicht porösem Glas bestehen kann (DE-A 31 05 768). Dabei liegt jedoch weder die Porenstruktur des erfindungsgemäßen Trägermaterials vor, noch ist an eine Verwendung in einem Wirbelbett-Bioreaktor und die dabei erzielbaren besonderen Wirkungen und Vorteile gedacht worden.

Die zu wählenden Porendurchmesser der erfindungsgemäßen offenporigen kugelförmigen Sinterkörper hängen sowohl von der Größe und Form der Mikroorganismen und/oder tierischen Zellen als auch von den angestrebten Bioreaktionen ab. Um den Mikroorganismen und/oder tierischen Zellen ein ungestörtes Wachstum zu ermöglichen, betragen die Porendurchmesser im allgemeinen mehr als 10 $\mu$m, meist sogar mehr als 100 $\mu$m. In Abhängigkeit von der Struktur der sich vermehrenden Mikroorganismen sind gelegentlich auch wesentlich höhere Porendurchmesser erforderlich.

Das offene Porenvolumen liegt im allgemeinen zwischen 40 und 75 Vol-%. Bei einem Porenvolumen von über 75 % nimmt die mechanische Festigkeit sehr rasch ab.

Die benötigte Größe der kugelförmigen Sinterkörper des erfindungsgemäßen Trägermaterials hängt von dem angestrebten biotechnischen Verfahren ab. Übliche Kugelgrößen liegen zwischen 1 und 10 mm Durchmesser.

Das erfindungsgemäße Trägermaterial ist besonders für den aeroben Abbau mit starkem mikrobiellem Wachstum geeignet, insbesondere bei der Reinigung von kommunalen und industriellen Abwässern. Dort sind die Verstopfungsprobleme besonders schwerwiegend. So zeigt sich bei der Verwendung von bekannten Festbettreaktoren, daß unabhängig von der Art des verwendeten Trägermaterials der Reaktor nach kurzer Zeit verschleimt. Die Mikroorganismen setzen sich an der Oberfläche fest und verhindern ein Einwandern in das Trägerinnere. Es ist deshalb versucht worden, kommunale Abwässer im Wirbelbett mit Trägermaterial aus offenporigem Sinterglas in Form von Raschigringen biotechnisch zu reinigen (DE-OS 34 10 650). Diese Versuche führten nur teilweise zum Erfolg. Wie beim Festbettreaktor verschleimt der innere Hohlraum des Raschigringes vollständig, und die äußere Oberfläche teilweise. Hierdurch ändert sich die gesamte Dichte des Raschigringes, und er wird ausgetragen. Ein kontinuierlicher Betrieb des Wirbelbettreaktors ist somit nicht möglich. Im Gegensatz dazu werden bei Verwendung des erfindungsgemäßen Trägermaterals im Wirbelbett die aus dem Inneren der Kugeln herausgewachsenen und an der Oberfläche befindlichen Mikroorganismen abgeschert, und die abgescherte Biomasse wird ausgetragen; es stellt sich somit im Wirbelbettreaktor eine im wesentlichen konstante Menge an lebender Biomasse ein. Da im Inneren der Kugeln die Vermehrung ungestört ist, können auch langsam wachsende Mikroorganismen sich vermehren und wirksam werden, da sie nicht mehr ausgetragen werden. Somit können bei Verwendung des erfindungsgemäßen Trägermaterials in einem Wirbelbettreaktor auch schwer abbaubare Stoffe zusätzlich abgebaut werden.

Die Verwendung des erfindungsgemäßen Trägermaterials ist nicht auf die Reinigung kommunaler und industrieller Abwässer bestimmt; das Trägermaterial kann auch zur biotechnischen Gewinnung von ernährungsessentiellen und pharmakologischen Substanzen und von Gärungsprodukten eingesetzt werden, wobei sich die beschriebenen Vorteile in entsprechender Form einstellen. Hier wie auch bei anderen Verwendungsarten zeigt sich als weiterer Vorteil der Verwendung des verwendungsgemäßen Trägermaterials, daß die Mikroorganismen unter günstigen Bedingungen immobilisiert und vermehrt werden, und daß die Reaktionsprodukte leicht abgetrennt werden können.

Es hat sich überraschenderweise gezeigt, daß es durch entsprechende Auswahl der Porenform und des Kugeldurchmessers möglich ist, in dem erfindungsgemäßen Trägermaterial gleichzeitig aerobe und anaerobe Verhältnisse einzustellen, derart, daß in einem aeroben Medium der Sauerstoffgehalt in den Kugeln bei Anwesenheit von Mikroorganismen oder tierischen Zellen von außen nach innen so stark abnimmt, daß in einer äußeren Kugelzone aerobe und in einer inneren Kugelzone anaerobe Bedingungen vorliegen. Ein solches gleichzeitiges Vorliegen von aeroben und anaeroben Bedingungen ist für viele biotechnische Verfahren von großer Bedeutung, insbesondere für den Abbau von Nitrit- und Nitrat-Gehalten zu molekularem Stickstoff in Wasser.

In diesem Zusammenhang hat sich gezeigt, daß das erfindungsgemäße Trägermaterial mit großem Vorteil bei der Wasserreinigung von Aquarien verwendet werden kann.

Bei dem erfindungsgemäßen Trägermaterial kann die mechanische Festigkeit der kugelförmigen Sinterkörper gegen Abrieb dadurch erhöht werden, daß die Oberfläche unter Erhalt der Offenporigkeit mit einer dünnen Kunststoffschicht beschichtet ist. Insbesondere Sinterkörper aus Sinterglas können dadurch wesentlich unempfindlicher gegen Stoßbelastung gemacht werden, da der Kunststoff einen hohen Anteil der Stoßenergie aufnimmt.

Je nach Art der Mikroorganismen und tierischen Zellen, aber auch in Abhängigkeit von der Bioreaktion selbst kann es nötig sein, die innere Oberfläche unter Beibehaltung der Offenporigkeit mit anorganischen und/oder organischen Schichten zu versehen. Solche Beschichtungen kennen sowohl dazu dienen, die Immobilisierung der Mikroorganismen und tierischen Zellen zu verbessern, als auch eine Absorption von Gasen oder anderen Stoffen zu ermöglichen.

Zur Herstellung des erfindungsgemäßen Trägermaterials wird zunächst ein Pulvergemisch aus einem feinkörnigen sinterfähigen Material und einer grobkörnigen, aus dem Sinterprodukt herauslösbaren Substanz, deren Schmelzpunkt höher liegt als die Sintertemperatur des sinterfähigen Materials, hergestellt. Das Pulvergemisch wird unter Zugabe eines Bindemittels in einem Granulierer zu Kugeln des gewünschten Durchmessers aufgranuliert. Nach dem Sintern und Abkühlen wird die Herauslösbare Substanz ausgewaschen.

Beispielsweise wird für die Herstellung von offenporigen Sinterglaskugeln aus Borosilikatglas (Glastyp 8330 der Firma SCHOTT GLASWERKE) und $K_2SO_4$ ein Gemisch aus 8330- Glas und Kaliumsulfat mit wässriger Tylose-Lösung versetzt. In einem Mischgranulierer wird solange granuliert, daß die Kugeln die gewünschte Größe erreichen. Dann wird getrocknet und anschließend gesintert. Die versinterten Kugeln werden je nach Anwendung fraktioniert und dann ausgewaschen.

Für die Herstellung von offenporigen Sinterglaskugeln aus Kalk-Natron-Silikatglas (Fensterglas) und NaCl wird ein Gemisch aus Fensterglas und NaCl mit einer mit NaCl gesättigten Tylose-Lösung versetzt. Das Granulieren, Trocknen und Sintern erfolgt ähnlich wie oben angegeben.

Die Herstellung der kugelförmigen Sinterkörper des erfindungsgemäßen Trägermaterials durch Granulieren, Trocknen und Sintern ist erheblich einfacher als die Herstellung von Sinterkörpern komplizierterer Gestalt, beispielsweise in Form von Raschigringen. Die Dichte bzw. das offene Porenvolumen der kugelförmigen Sinterkörper kann bei der Herstellung durch entsprechende Wahl des Gewichtsverhältnisses von sinterfähigem Material zu herauslösbarer Substanz eingestellt werden, während die Porengröße durch entsprechende Wahl der Korngröße der herauslösbaren Substanz bestimmt wird. Als herauslösbare Substanzen kommen in erster Linie natürlich vorkommende preiswerte wasserlösliche Salze in Frage, insbesonder NaCl und $K_2SO_4$. Da die Sintertemperatur des sinterfähigen Materials niedriger liegen soll als der Schmelzpunkt der herauslösbaren Substanz, eignen sich hierzu in erster Linie Gläser oder ein Gemisch aus natürlichen Mineralien und niedrig schmelzenden Gläsern als Bindemittel.

Das erfindungsgemäße Trägermaterial kann in Fest- oder Wirbelbett-Reaktoren verwendet werden. Besonders vorteilhaft ist eine Verwendung in einem Festbett-Reaktor, in dem zur Reinigung der Kugeloberflächen von Mikroorganismen und tierischen Zellen vorübergehend wirbelbettähnliche Bedingungen eingestellt werden können. Auf diese Weise kann die Oberfläche des Trägermaterials gereinigt und das entfernte Material mit der flüssigen Phase des Reaktors abgeführt werden, ohne daß der Reaktor geöffnet werden muß.

Das erfindungsgemäße Trägermaterial kann sowohl für aerobe als auch für anaerobe Prozesse verwendet werden. Diese beiden Prozesse unterscheiden sich bezüglich der Verstopfung nicht grundsätzlich, sondern nur in der Geschwindigkeit, mit der sich die Verstopfung aufbaut.

Ausführungsbeispiele

Beispiel 1:

In vergleichenden Untersuchungen wurden offenporige Sinterglaskörper in einem Tauchkörperreaktor, Festbettumlaufreaktor und Wirbelbettreaktor untersucht.

Das offenporige Sinterglas wurde im Tauchkörperreaktor in Form von Rohren, im Festbettreaktor in Form von Raschigringen und im Wirbelbettreaktor in Form von Raschigringen und Kugeln eingesetzt. Die Rohre und Raschigringe wurden durch Mischen von 40 Gew.-% Glaspulver und 60 Gew.-% Salzpulver, Extrudieren bzw. Verpressen, Versintern und Auswaschen des Salzes hergestellt. Bei dem Glas handelt es sich, wie in Beispiel 1, um das Borosilikatglas von SCHOTT GLASWERKE, Typ 8330, und bei dem Salz um $K_2SO_4$.

In den Untersuchungen wurden die Immobilisierung von Mikroorganismen im Zeitablauf und die Abbauleistung der Mikroorganismen untersucht. Die angebotene Oberfläche des Trägermaterials betrug jeweils 0.7 $m^2$/l Reaktorvolumen. Im Versuchsverlauf wurde ein synthetisches Abwasser mit einem chemischen Sauerstoffbedarf von 1000 mg/l eingesetzt. Die Reaktoren wurden mit dem gleichen Luftdurchsatz beaufschlagt. Zunächst ist die Immobilisierung bei einer hydraulischen Verweilzeit von 10 h beobachtet worden, gefolgt von einer Periode mit 6 h Verweilzeit.

Die rohrförmigen Träger im Tauchkörperreaktor und die Raschigringe im Festbettreaktor waren nach kurzer Zeit vollständig bewachsen, da sich die Aerobier schnell vermehren.

Im Wirbelbettreaktor war die von der Geometrie der Raschigringe herrührende zentrale Bohrung nach einiger Zeit mit Biomasseflocken gefüllt. Die niedrige Turbulenz in der Bohrung reicht aus, um eine Verstopfung zu bewirken Durch die Scherkräfte im Wirbelbettstrom waren die Kanten der Raschigringe abgerundet und die äußere Oberfläche weitgehend frei von Mikroorganismen. Die Biomasseflocken in der Bohrung der Raschigringe reichen aus, um die Raschigringe im Wirbelbettstrom mitzureißen und auszutragen. Weiterhin verhindert die Biomasse in der Bohrung eine freie Durchströmung des Trägermaterials, so daß an der Oberfläche der Bohrung keine aerobe Umsetzung stattfinden kann.

Nur die obersten Schichten des mikrobiellen Bewuchses der äußeren Oberfläche des Raschigringes tragen zur reinen aeroben Umsetzung bei. Da aber an der Gesamtmineralisierung auch semiaerobe und anaerobe Reaktionsschritte beteiligt sein können, kann die Sauerstofflimitierung ab einer gewissen Porentiefe toleriert werden.

Bei einer Porenweite von 60 bis 300 $\mu$m und einem offenen Porenvolumen von 55 bis 65 Volumen-% betrug die Tiefe des Bewuchses von Mikroorganismen in den Porenräumen 2 - 3 mm. Bis zu diesen Tiefen findet somit eine Mineralisierung statt.
Die Kugeln mit einem Durchmesser von 5 bis 6 mm zeigten im Wirbelschichtreaktor nur in den Porenräumen Biomassenaufwuchs. Nachwachsende, d.h. aus den Poren an die Oberfläche der Kugeln austretende Mikroorganismenteile wurden abgeschert. Nach Bewuchs mit Mikroorganismen geht durch die Elastizität der Biomasse der Abrieb des Trägermaterials drastisch zurück.

Die Abbauleistung der auf den kugelförmigen Trägerkörpern im Wirbelbettreaktor immobilisierten Mikroorganismen war gegenüber dem Festbettumlaufreaktor um den Faktor 1.5 und gegenüber dem Tauchkörperreaktor um den Faktor 1.8 erhöht.

Beispiel 2:

Die Immobilisierung von mycelbildenden Bakterien zur Produktion von Antibiotika, wie beispielsweise von Streptomyces tendae, bereitete an offenporigem, kugeligem Sinterglasträgermaterial einige Schwierigkeiten. Die Bakterien wuchsen zwar auf der angebotenen Oberfläche, jedoch gab es keinen zusammenhängenden Belag. Die Porenräume waren nur gelegentlich bewachsen.

Eine deutlich Erhöhung des Bewuchses konnte durch spezifische Beschichtungen erreicht werden. Im Falle von Streptomyces tendae war beispielsweise die Beschichtung mit Silanen, die Amino- bzw. Mercaptogruppen enthalten, am erfolgreichsten.

Ebenfalls von Bedeutung ist die Form und der physiologische Zustand der Biomasse, die zum Bewachsen der Träger verwendet werden. Sie entscheidet über die Bewuchsdichte im Porenraum, die wiederum im Wirbelbett den entscheidenden Beitrag zur Produktivität leistet, da ein Teil der oberflächlich wachsenden Biomasse abgeschert wird.

Da bei der Produktion von Sekundärmetaboliten (als was Antibiotika stoffwechseltechnisch anzusehen sind), eine Entkopplung von Wachstum und Produktivität zu einem hohen Prozentsatz gegeben ist, spielt das Abscheren der Biomasse nicht eine so überragende Rolle wie bei wachstumsgekoppelten Prozessen. Überragenden Einfluß haben hier die definierten Stofftransportlimitierungen, die für die bewachsenen Poren in Kugeln verschiedenen Durchmessers vorgegeben werden können.

## Patentansprüche

1. Verfahren zur Durchführung biotechnologischer Prozesse mit Bioreaktoren im Fest- oder Wirbelbettbetrieb unter Verwendung poröser Sinterkörper aus offenporigem anorganischem Sintermaterial, vorzugsweise Sinterglas, welches im Inneren durchgehende, nach außen offene Poren von einer Größe aufweist, die ein Mehrfaches der Größe der Mikroorganismen oder tierischen Zellen beträgt, wobei die Poren einen freien Flüssigkeits- und Gasaustausch mit dem Inneren des Trägermaterials erlauben, dadurch gekennzeichnet, daß kugelförmige Sinterkörper verwendet werden und im Wirbelbettbetrieb die Scherkräfte an der Oberfläche der Trägerkörper über die geeignete Wahl der Dichte und des Durchmessers der Trägerkörper so eingestellt werden, daß das Wachstum der Mikroorganismen auf das Innere der Trägerkörper beschränkt bleibt.

2. Verfahren nach Anspruch 1, dadurch gekennzeichnet, daß der Durchmesser und die Porenstruktur der Kugeln so gewählt werden, daß in einem aeroben Medium der Sauerstoffgehalt in den Kugeln bei Anwesenheit von Mikroorganismen oder tierischen Zellen von außen nach innen so stark abnimmt, daß in einer äußeren Kugelzone aerobe und in einer inneren Kugelzone anaerobe Bedingungen vorliegen.

3. Verfahren nach Anspruch 1 oder 2, dadurch gekennzeichnet, daß zur Erhöhung der Abriebsfestigkeit an ihrer Oberfläche unter Erhalt der Offenporigkeit mit Kunststoff beschichtete Trägerkörper verwendet werden.

4. Verfahren nach Anspruch 1 oder 2, dadurch gekennzeichnet, daß zur Verbesserung der Immobilisierung der Mikroorganismen oder tierischen Zellen mit anorganischen und/oder organischen Schichten unter Erhalt der Offenporigkeit versehene Trägerkörper verwendet werden.

5. Verfahren nach einem der vorhergehenden Ansprüche, dadurch gekennzeichnet, daß ein Festbett-Durchlaufreaktor eingesetzt wird, der zur Entfernung der an den Kugeloberflächen herauswachsenden Mikroorganismen und tierischen Zellen vorübergehend im Wirbelbett-Betrieb gefahren wird.

6. Verfahren nach den Ansprüchen 1 bis 4, dadurch gekennzeichnet, daß der biotechnologische Prozeß unter anaeroben Bedingungen abläuft.

7. Verfahren nach den Ansprüchen 1 bis 4, dadurch gekennzeichnet, daß der biotechnologische Prozeß unter aeroben Bedingungen abläuft.

8. Verfahren nach den Ansprüchen 1 bis 4, dadurch gekennzeichnet, daß der biotechnologische Prozeß unter gemischten aeroben und anaeroben Bedingungen abläuft.

9. Verfahren nach einem der Ansprüche 1 bis 5, dadurch gekennzeichnet, daß die Bioreaktoren mit kommunalen und industriellen Abwässern beschickt werden.

10. Verfahren nach einem der Ansprüche 1 bis 5, dadurch gekennzeichnet, daß die Bioreaktoren mit nitrit- und nitrathaltigen Wässern beschickt werden.

11. Verfahren nach einem der Ansprüche 1 bis 5, dadurch gekennzeichnet, daß die Bioreaktoren mit verunreinigten Wässern aus Aquarien beschickt werden.

12. Verfahren nach den Ansprüchen 1 bis 4, dadurch gekennzeichnet, daß solche Mikroorganismen eingesetzt werden, die ernährungsessentielle und/oder pharmakologische Substanzen und/oder Gärungsprodukte an das flüssige Medium abgeben.

## Claims

1. Method of carrying out biotechnological processes with bioreactors in fixed or fluidized bed operation, using porous sintered bodies of open-pored inorganic sintered material, preferably sintered glass which has going right through the interior outwardly open pores of a size which is a multiple of the size of the microorganisms or animal cells, the pores permitting a free liquid and gas exchange with the interior of the carrier material, characterized in that spherical sintered bodies are used and in fluidized bed operation the shearing forces at the surface of the support bodies are set by suitable choice of the density and the diameter of the support bodies such that the growth of the microorganisms remains restricted to the interior of the support bodies.

2. Method according to Claim 1, characterized in that the diameter and the pore structure of the spheres are selected such that in an aerobic medium the oxygen content in the spheres drops sharply from the outside to the inside in the presence of microorganisms or animal cells such that in an outer sphere zone aerobic conditions are present and in an inner sphere zone anaerobic conditions are present.

3. Method according to Claim 1 or 2, characterized in that to increase the abrasion resistance at their surface while maintaining the open-pored nature, support bodies coated with plastics are used.

4. Method according to Claim 1 or 2, characterized in that to improve the immobilization of the microorganisms or animal cells support bodies provided with inorganic and/or organic layers while maintaining the open-pored nature are used.

5. Method according to one of the preceding claims, characterized in that a fixed bed continuous reactor is used which is operated temporarily in fluidized bed operation to remove the microorganisms and animal cells growing out of the sphere surfaces.

6. Method according to Claims 1 to 4, characterized in that the biotechnological process takes place under anaerobic conditions.

7. Method according to Claims 1 to 4, characterized in that the biotechnological process takes place under aerobic conditions.

8. Method according to Claims 1 to 4, characterized in that the biotechnological process takes place under mixed aerobic and anaerobic conditions.

9. Method according to one of Claims 1 to 5, characterized in that the bioreactors are charged with community and industrial effluents.

10. Method according to one of Claims 1 to 5, characterized in that the bioreactors are charged with water containing nitrites and nitrates.

11. Method according to one of Claims 1 to 5, characterized in that the bioreactors are charged with soiled water from aquaria.

**12.** Method according to Claims 1 to 4, characterized in that those microorganisms are used which give off substances essential for nutrition and/or pharmacological substances and/or fermentation products to the liquid medium.

**Revendications**

**1.** Procédé de mise en oeuvre de processus biotechnologiques avec des bioréacteurs en mode à lit fixe ou à lit fluidisé, en utilisant des corps frittés poreux en matériau de frittage inorganique à pores ouverts, de préférence du verre fritté, qui présente à l'intérieur des pores continus, ouverts vers l'extérieur, d'une dimension qui est un multiple de la dimension des microorganismes ou cellules animales, les pores permettant le libre échange de liquides et de gaz à l'intérieur du matériau support, caractérisé par le fait que l'on utilise des corps frittés sphériques et qu'en mode à lit fluidisé les forces de cisaillement à la surface des corps supports sont réglées, par le choix approprié de la densité et du diamètre des corps supports, de manière telle que la croissance des microorganismes soit limitée à l'espace intérieur des corps supports.

**2.** Procédé selon la revendication 1, caractérisé par le fait que le diamètre et la structure des pores des sphères sont choisis tels que dans un milieu aérobie, la teneur en oxygène dans les sphères diminue, en présence de microorganismes ou de cellules animales, de l'extérieur vers l'intérieur si fortement qu'il existe des conditions aérobies dans une zone extérieure de la sphère et des conditions anaérobies dans une zone intérieure de la sphère.

**3.** Procédé selon la revendication 1 ou 2, caractérisé par le fait que, en vue de l'augmentation de leur résistance à l'abrasion, on utilise des corps supports recouverts de matière plastique à leur surface, en maintenant les pores ouverts.

**4.** Procédé selon la revendication 1 ou 2, caractérisé par le fait que, en vue d'une meilleure immobilisation des microorganismes ou des cellules animales, on utilise des corps supports recouverts de couches inorganiques et/ou organiques, en maintenant les pores ouverts.

**5.** Procédé selon l'une des revendications précédentes, caractérisé par le fait que l'on utilise un réacteur continu à lit fixe qui fonctionne temporairement en mode à lit fluidisé en vue d'éliminer les microorganismes et cellules animales sortant aux surfaces de la sphère.

**6.** Procédé selon les revendications 1 à 4, caractérisé par le fait que le processus biotechnologique se déroule dans des conditions anaérobies.

**7.** Procédé selon les revendications 1 à 4, caractérisé par le fait que le processus biotechnologique se déroule dans des conditions aérobies.

**8.** Procédé selon les revendications 1 à 4, caractérisé par le fait que le processus biotechnologique se déroule dans des conditions aérobies et anaérobies mixtes.

**9.** Procédé selon l'une des revendications 1 à 5, caractérisé par le fait que les bioréacteurs sont chargés avec des eaux usées communales et industrielles.

**10.** Procédé selon l'une des revendications 1 à 5, caractérisé par le fait que les bioréacteurs sont chargés avec des eaux contenant des nitrites et des nitrates.

**11.** Procédé selon l'une des revendications 1 à 5, caractérisé par le fait que les bioréacteurs sont chargés avec des eaux sales provenant d'aquariums.

**12.** Procédé selon les revendications 1 à 4, caractérisé par le fait que l'on utilise des microorganismes qui transmettent au milieu liquide des substances alimentaires essentielles et/ou pharmacologiques et/ou des produits de fermentation.